# EUROPEAN PATENT APPLICATION

(11) **EP 4 799 674 A1**
(43) Date of publication of application: **02.09.2026**
(21) Application number: 26160630.5
(22) Date of filing: 25.02.2026
(51) Int. Cl.: A61M 60/122, A61M 60/859, A61M 60/878, H01R 13/52, H01R 13/58, H01R 24/58

(54) **IMPLANTABLE ASSEMBLY COMPRISING AN IMPLANTABLE MEDICAL LEAD AND A STIFFENING MEMBER FOR STIFFENING A PART OF THIS MEDICAL LEAD**

(30) Priority: 28.02.2025 FR 2502044
(71) Applicant: Fineheart, 33600 Pessac (FR)
(72) Inventor: COLLAS, Jérémy, 33700 Mérignac (FR); PEYRE, André, 19130 Objat (FR); GUTTON, Benjamin, 33700 Mérignac (FR)
(74) Representative: Plasseraud IP

(57) **Abstract**

The present invention relates to an implantable assembly comprising an implantable medical lead (13) having, at a first end, at least one lead connector (14) intended to be received in a connection housing of a connector head (10) for an implantable medical device (11) in order to form a connection to the latter, said connector head (10) having an inlet port defining an interior passage (17) in communication with said connection housing for the insertion of said at least one lead connector (14) into the connection housing, said assembly also comprising a stiffening member (18), for stiffening the implantable medical lead (13), mounted on said first end upstream of said at least one lead connector (14) in order to stiffen the part of this implantable medical lead (13) positioned in line with this stiffening member (18), said stiffening member being further configured to be partially inserted into said interior passage (17), closing at least the inlet of the latter so as to prevent the passage of body fluid, in particular blood, into the connection housing.

## Description

### Technical field

The present invention relates to the field of implantable medical leads for their use in implantable devices such as ventricular assist devices (VAD), probes, etc.

It relates more particularly to an implantable assembly comprising such an implantable medical lead and a stiffening member for stiffening a part of this medical lead at the level of its connection to an implantable control unit.

### Prior art

Heart failure (HF) is a pathological state in which a patient's heart is unable to provide a rate of blood flow necessary for the metabolic needs of the body.

It is known to treat heart failure by implanting a ventricular assist device (VAD), which is an artificial heart pump.

This mechanical pump does not replace the heart, which continues to function, but instead helps the weakened ventricle to increase the rate of blood flow in such a way as to meet the needs of the individual.

This assistance may be temporary while waiting for an organ to become available for a heart transplant.

However, a significant proportion of patients will not receive such a transplant, either because they may not be candidates for transplantation, for example because of severe heart failure, or because no suitable transplant is available for these patients.

In that case, ventricular assistance is used as the specific solution, i.e. the artificial heart pump is implanted for the long term.

These heart pumps are therefore the subject of intense research aimed at improving the quality of life of patients with heart failure, it being possible for these pumps to remain active for several months to several years.

A great deal of progress has been made, and today we have ventricular assist devices that are more compact and quieter and that provide an increased service life.

The implantable heart pumps of the prior art are thus typically equipped with an integrated electric motor to ensure their operation, with the speed of rotation of the pump providing the force necessary for circulating the blood from the weakened ventricle to the circulatory system.

Moreover, such a heart pump is typically controlled by a control unit implanted entirely within the patient's body, for example in the epigastric region, in the upper part of the abdomen.

Such implantation without a percutaneous transmission line avoids the risk of infection, which is the primary cause of complications and mortality in patients.

The motorization of this heart pump is thus connected by wire to the implanted control unit in order to receive control signals.

This control unit is also connected by a wired link to a cardiac activity acquisition probe, in order to synchronize the operation of the pump with the ventricular systolic activity and thereby support the natural functioning of the blood flow.

This control unit can also have a wired connection to other pieces of medical equipment, such as an implantable defibrillator, etc.

Each wired link connecting the control unit to these implantable pieces of equipment consists of an implantable medical lead having, at a first end, a set of lead connectors intended to cooperate with a plurality of head connectors positioned in a connection housing of a connector head of the control unit, so as to form a connection between this control unit and the implantable medical lead.

However, infiltration of blood into these connection housings has been observed, leading to an accumulation of blood which, when it hardens, binds the set of lead connectors in its connection housing and prevents withdrawal of the lead connectors.

When changing an implantable medical lead, it is thus necessary to use pliers to pull the end of this medical lead and attempt to extract the latter from the body of the control unit.

Generally, the latter is caused to break because of excessive bending applied at the junction between cable and connector assembly.

The maintenance of an implantable medical connection is therefore a delicate and difficult operation for a practitioner.

In addition, when a detached segment of the implantable medical lead remains wedged in its connection housing, its recovery is a time-consuming task. Object of the invention

The object of the present invention is to overcome the drawbacks of the prior art and to respond to the above-mentioned constraints by proposing an implantable assembly which is simple in its design and in its mode of operation, ensuring simple and safe disconnection of an implantable medical lead connected to an implanted control unit.

Another object of the present invention is to provide an implantable assembly of this kind that is economical and particularly reliable.

### Summary of the invention

To this end, the invention relates to an implantable assembly comprising an implantable medical lead having, at a first end, at least one lead connector intended to be received in a connection housing of a connector head for an implantable medical device in order to form a connection to the latter, the connector head having an inlet port defining an interior passage in communication with the connection housing for the insertion of said at least one lead connector into the connection housing, this assembly also comprising a stiffening member, or stiffening element, for stiffening the implantable medical lead, mounted on the first end upstream of said at least one lead connector in order to stiffen the part of this implantable medical lead positioned in line with this stiffening member, this stiffening member being further configured to be partially inserted into the interior passage, closing at least the inlet of the latter so as to prevent the passage of body fluid, in particular blood, into the connection housing.

An implantable assembly of this kind is particularly suitable when connecting an implanted heart pump to a beating heart or a non-beating heart benefiting from extracorporeal circulation, because it is particularly simple to use.

Advantageously, an implantable assembly of this kind provides increased convenience for the surgical team during the maintenance of an implantable medical connection comprising an implanted control unit and equipment such as a heart pump, connected to this control unit.

Since the implantable medical lead comprises an outer sheath over part of its length, this stiffening member is mounted at least partially, or even totally, on this outer sheath in order to leave free the set of lead connectors positioned at the free end of the implantable medical lead. The connection between the medical lead and the medical device is thus not altered.

Furthermore, the stiffening of the implantable medical lead at this first end prevents breakage of this medical lead.

Furthermore, it is advantageously observed that the medical lead is firmly held at its connection and that the latter is impervious to the blood surrounding the implanted medical device.

This medical lead can thus be a cable typically used to convey cardiac and electrical signals, or signals indicative of parameters of a heart pump and/or its components, or signals of a state of charge of the battery supplying the heart pump and/or its accessories.

According to a particular embodiment of this implantable assembly, at least one part of this stiffening member intended to be positioned outside the connector head, and situated in the continuation of the portion of the stiffening member intended to be inserted into the interior passage, forms a mechanical stop by which the introduction of said stiffening member into said interior passage is limited to this portion alone, while allowing this stiffening member to be gripped by a gripping tool, in order to ensure its withdrawal from the interior passage upon disconnection of the implantable medical lead.

Such a configuration of the stiffening member facilitates the disconnection of the implantable medical lead from the medical device by use of a gripping tool, such as pliers.

Preferably, the outer surface of said at least one part of this stiffening member intended to be positioned outside the connector head has a smooth surface in order to prevent its colonization by cells.
Preferably, this smooth surface has an arithmetic mean roughness Rz of less than or equal to 5 µm, and better still less than or equal to 3 µm.

According to another embodiment of this implantable assembly, said at least one part of this stiffening member intended to be positioned outside the connector head has a face shaped to be pressed flat against the body of the connector head when the first end of said implantable medical lead is plugged into the connector head.

According to yet another embodiment of this implantable assembly, said stiffening member is a single piece or made in one piece.
Preferably, it is made of a single material. For illustrative purposes only, this stiffening member is made of ceramic or of a thermoplastic material such as polyetheretherketone (PEEK), polyetherimide (PEI) or polyphenylsulfone (PPSU). Advantageously, these materials are insulating in order to provide electrical insulation, for example, if the sheath surrounding the cable breaks.

According to yet another embodiment of this implantable assembly, said stiffening member is configured to be joined to said implantable medical lead by interlocking.
Preferably, this stiffening member comprises a central lumen in order to be force-fitted onto this outer sheath and thus to be joined to the implantable medical lead.
Alternatively or in addition, the stiffening member is assembled on the implantable medical lead by adhesive bonding using an appropriate glue.

According to yet another embodiment of this implantable assembly, said stiffening member comprises a central lumen for the passage of the implantable medical lead.

According to yet another embodiment of this implantable assembly, with said interior passage being tubular, or substantially tubular, at least the portion of the stiffening member intended to be inserted into the interior passage is an elongate tubular body having a diameter greater than or equal to the diameter of said interior passage, or an elongate tubular body provided with radial flanges which are spaced apart from one another, are of identical geometry and have a diameter greater than or equal to the diameter of said interior passage.

The present invention also relates to a stiffening member for an implantable assembly as described above, this stiffening member being configured to form an obturator for an inlet port of a connector head for an implantable medical device, said obturator comprising an obturator head continued by a cylindrical body, a lumen passing through this obturator for the passage of an implantable medical lead, this obturator head being shaped to be gripped by a gripping tool such as pliers.

The present invention also relates to an implantable medical connection comprising a cardiac activity acquisition probe and an implantable medical device comprising at least two connector heads, each one comprising a connection housing, the end of an implantable medical lead comprising a set of lead connectors being plugged into one of said connector heads in order to form a connection to this implantable medical device, the other end of said implantable medical lead being connected to said cardiac activity acquisition probe, each connector head comprising an inlet port defining an interior passage in communication with its connection housing, a stiffening device being mounted on said guide wire, said guide wire and said stiffening member belonging to the implantable assembly as described above.

Preferably, this connection further comprises another implantable medical lead connecting a heart pump to said implantable medical device and also another medical lead connecting a power source to this implantable medical device, each implantable medical lead being integral with a stiffening member belonging to the implantable assembly as described above. This implantable medical device advantageously comprises a processor and a set of software instructions which, when executed by this processor, make it possible to implement a method of controlling this heart pump, this heart pump being intended to deliver blood under pressure at a flow rate proportional to the speed of rotation Vᵣₚₘ of this pump.

More generally, the present invention also concerns an implantable assembly comprising an implantable medical lead having, at a first end, at least one lead connector intended to be received in a connection housing of a connector head for an implantable medical device in order to form a connection with the latter, the connector head having an entry port defining an interior passage in communication with the connection housing for insertion of said at least one lead connector into the connection housing, said assembly also comprising a stiffening member, or mechanical reinforcement member, which is added to the mechanical lead and is removable from this medical lead, said stiffening member being mounted on the first end upstream of said at least one lead connector in order to stiffen the part of this implantable medical lead positioned in line with this stiffening member and to facilitate the introduction of the medical lead using a gripping tool such as pliers, capable of exerting pressure, this stiffening member being further configured to be partially inserted into the interior passage, sealing at least the inlet of the latter so as to prevent the passage of body fluid, in particular blood, into the connection housing.

### Brief description of the drawings

Further advantages, aims and particular features of the present invention will become apparent from the following description, which is given for nonlimiting and explanatory purposes and with reference to the appended drawings, in which:
**Fig. 1**

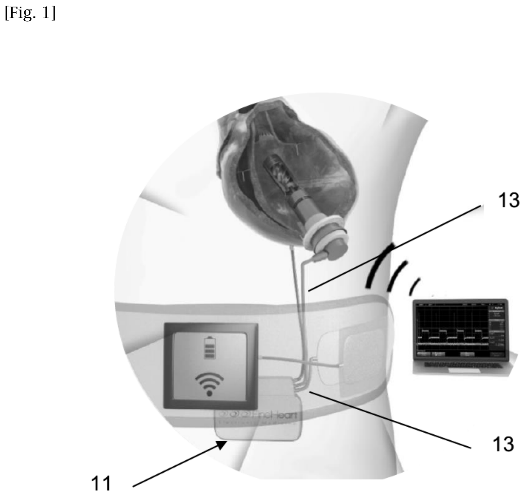

   [Fig. 1] is a simplified view of an implantable medical connection according to a particular embodiment of the present invention;
**Fig. 2**

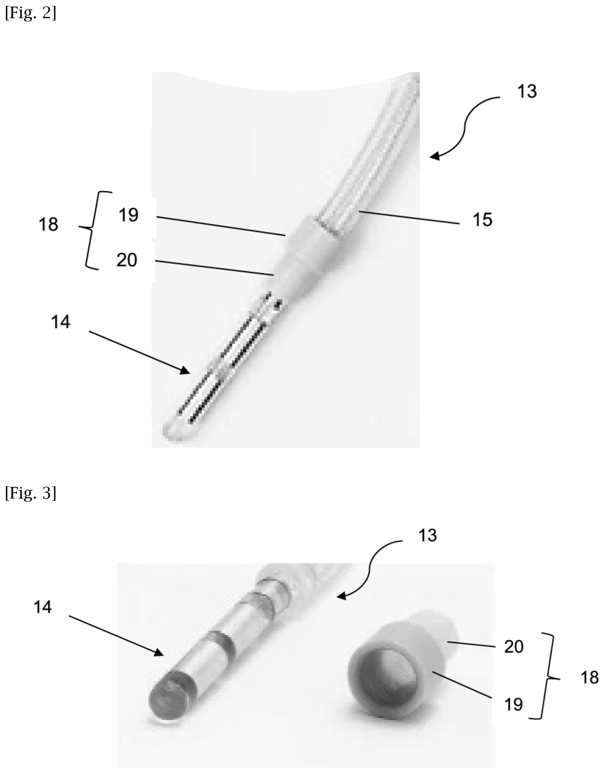

   [Fig. 2] is a partial top view of an implantable assembly according to a particular embodiment of the present invention;
**Fig. 3**
   [Fig. 3] is a partial view of the implantable assembly from Fig.2, with the stiffening member and the implantable medical lead separated;
**Fig. 4**

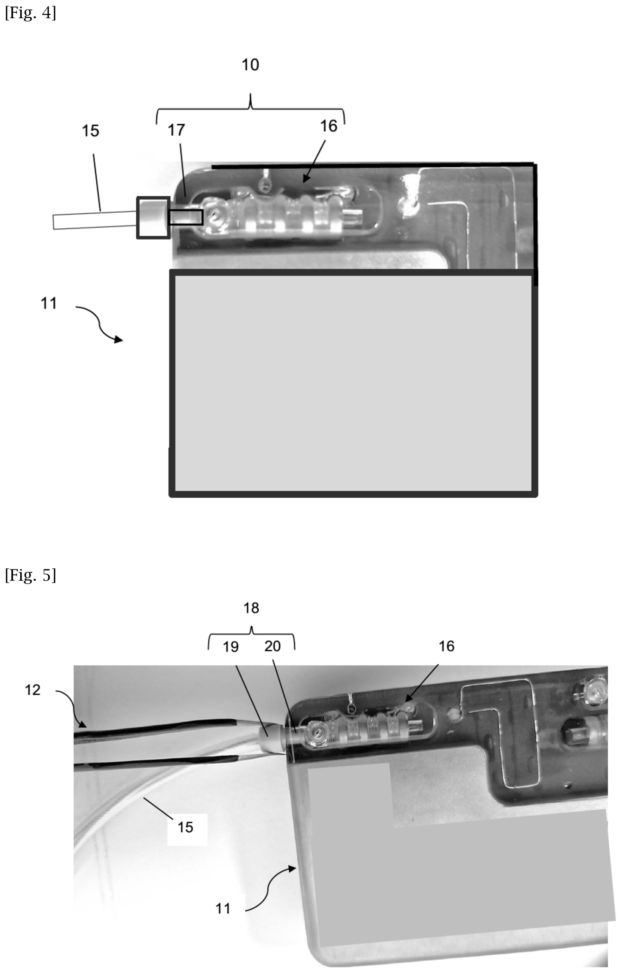

   [Fig. 4] is a partial schematic representation of a connector head of an implantable medical device into which an implantable assembly from Fig. 2 is connected;
**Fig. 5**
   [Fig. 5] shows the partial withdrawal, by means of pliers, of the implantable assembly illustrated in Fig. 2 from a connector head of a medical device.

### Description of the embodiments

The drawings and the description below contain, for the most part, elements of a certain nature. They can therefore serve not only to clarify the understanding of the present invention but also to contribute to the definition thereof, if necessary.

First of all, it is noted that the figures are not to scale.

Figures 2 to 5 schematically illustrate an implantable assembly according to a particular embodiment of the present invention.

This implantable assembly is shown, on the one hand, connected to a connector head 10 of an implantable medical device 11 such as a control unit, in Fig. 4, and, on the other hand, during disconnection by a practitioner using a gripping tool 12, here pliers, in Fig. 5.

This implantable assembly comprises an implantable medical lead 13 having, at a first end, a set 14 of lead connectors, and an outer tubular sheath 15 coaxially covering one or more electrical conductors connected to said lead connectors.

The set 14 of lead connectors is intended to be received in a connection housing 16 of a connector head 10 for an implantable medical device 11 in order to form a connection to the latter.

This connector head 10 comprises an input port defining an interior passage 17 in communication with this connection housing 16 for the insertion of this set 14 of lead connectors into this connection housing 16, so as to form the connection between the implantable medical lead 13 and the implantable medical device 11.

This implantable assembly also comprises a stiffening member 18, for stiffening the implantable medical lead 13, mounted on the first end upstream of the set 14 of lead connectors in order to stiffen the part of this implantable medical lead 13 positioned in line with this stiffening member 18.

This stiffening member 18 is made here in a single piece from polyetheretherketone (PEEK). Advantageously, this material is more flexible than titanium or epoxy, allowing its insertion by force.

A first part 19 of this stiffening member 18 is intended to be positioned outside the connector head 10, while a second part 20 of this stiffening member 18 is intended to be inserted into the interior passage 17.

The first part 19 of this stiffening member 18 forms a mechanical stop by which the introduction of this stiffening member 18 into the interior passage 17 is limited to this second part 20 alone.

It also allows this stiffening member 18 to be gripped by a gripping tool 12 such as pliers, so as to ensure its withdrawal from the interior passage 17 when the implantable medical lead 13 is disconnected.

With the interior passage 17 being tubular, the second part 20 of the stiffening member 18 has a cylindrical shape with a central tubular orifice for the passage of the implantable medical lead.

This stiffening member 18 is force-fitted onto the outer tubular sheath 15 of the implantable medical lead 13.

## Claims

1. Implantable assembly comprising an implantable medical lead (13) having, at a first end, at least one lead connector (14) intended to be received in a connection housing of a connector head (10) for an implantable medical device (11) in order to form a connection to the latter, the connector head (10) having an inlet port defining an interior passage (17) in communication with the connection housing for the insertion of said at least one lead connector (14) into the connection housing, said assembly also comprising a stiffening member (18), for stiffening the implantable medical lead (13), mounted on the first end upstream of said at least one lead connector (14) in order to stiffen the part of this implantable medical lead (13) positioned in line with this stiffening member (18), said stiffening member (18) being further configured to be partially inserted into said interior passage (17), closing at least the inlet of the latter so as to prevent the passage of body fluid, in particular blood, into the connection housing.

2. Implantable assembly according to Claim 1, **characterized in that** at least one part of this stiffening member (18) intended to be positioned outside the connector head (10), and situated in the continuation of the portion of the stiffening member (18) intended to be inserted into the interior passage (17), forms a mechanical stop by which the introduction of said stiffening member (18) into said interior passage (17) is limited to this portion alone, while allowing this stiffening member (18) to be gripped by a gripping tool (12), such as pliers, in order to ensure its withdrawal from the interior passage (17) upon disconnection of the implantable medical lead (13).

3. Implantable assembly according to Claim 2, **characterized in that** the outer surface of said at least one part of this stiffening member (18) intended to be positioned outside the connector head (10) has a smooth surface in order to prevent its colonization by cells.

4. Implantable assembly according to Claim 2 or 3, **characterized in that** said at least one part of this stiffening member (18) intended to be positioned outside the connector head (10) has a face shaped to be pressed flat against the body of the connector head (10) when the first end of said implantable medical lead (13) is plugged into the connector head (10).

5. Implantable assembly according to any one of the preceding claims, **characterized in that** said stiffening member (18) is made in one piece.

6. Implantable assembly according to the preceding claim, **characterized in that** said stiffening member (18) is made of ceramic or of a thermoplastic material.

7. Implantable assembly according to any one of the preceding claims, **characterized in that** said stiffening member (18) is configured to be joined to the implantable medical lead (13) by interlocking.

8. Implantable assembly according to any one of the preceding claims, **characterized in that** said stiffening member (18) comprises a central lumen for the passage of the implantable medical lead (13).

9. Implantable assembly according to any one of the preceding claims, **characterized in that**, with said interior passage (17) being tubular, or substantially tubular, at least the portion of the stiffening member (18) intended to be inserted into the interior passage (17) is an elongate tubular body having a diameter greater than or equal to the diameter of said interior passage (17), or an elongate tubular body provided with radial flanges which are spaced apart from one another, are of identical geometry and have a diameter greater than or equal to the diameter of said interior passage (17).

10. Stiffening member for an implantable assembly according to any one of Claims 1 to 9, **characterized in that** it is configured to form an obturator for an inlet port of a connector head (10) for an implantable medical device (11), said obturator comprising an obturator head continued by a cylindrical body, a lumen passing through this obturator for the passage of an implantable medical lead (13), this obturator head being shaped to be gripped by a gripping tool (12).
